# EUROPÄISCHE PATENTSCHRIFT

(11) **EP 4 370 650 B1**
(45) Veröffentlichungstag und Bekanntmachung des Hinweises auf die Patenterteilung: **16.04.2025**
(21) Anmeldenummer: 22748352.6
(22) Anmeldetag: 12.07.2022
(51) Int. Cl.: C12N 1/06, C12R 1/865

(54) **VERFAHREN ZUR ISOLIERUNG VON PROTEINEN UND SACCHARIDEN AUS HEFE**
METHOD FOR THE ISOLATION OF PROTEINS AND SACCHARIDES FROM YEAST
PROCÉDÉ D'ISOLEMENT DE PROTÉINES ET DE SACCHARIDES DE LA LEVURE

(30) Priorität: 12.07.2021 EP 21185098
(43) Veröffentlichungstag der Anmeldung: 22.05.2024
(73) Patentinhaber: Yeastup AG, 5200 Brugg (CH)
(72) Erfinder: RIEDL, Wolfgang, 79591 Eimeldingen (DE); VERPOORTEN, Rudi, 6332 Hagendorn-Cham (CH)
(74) Vertreter: Keller Schneider Patent- und Markenanwälte AG
(86) Internationale Anmeldenummer: PCT/EP2022/069504
(87) Internationale Veröffentlichungsnummer: WO 2023/285480

(56) Entgegenhaltungen:
- EP-A1- 0 950 716
- WO-A1-2006/121803
- WO-A1-2010/070207
- WO-A2-2020/127951
- SAOEWANEE THAMMAKITI ET AL: "Preparation of spent brewer' s yeast beta-glucans for potential applications in the food industry", INTERNATIONAL JOURNAL OF FOOD SCIENCE AND TECHNOLOGY, vol. 39, 1 January 2004 (2004-01-01), pages 21 - 29, XP055704411
- SILVA ARAÚJO VILMA BARBOSA DA ET AL: "Followed extraction of [beta]-glucan and mannoprotein from spent brewer's yeast (Saccharomyces uvarum) and application of the obtained mannoprotein as a stabilizer in mayonnaise", INNOVATIVE FOOD SCIENCE AND EMERGING TECHNOLOGIES, vol. 23, 1 June 2014 (2014-06-01), NL, pages 164 - 170, XP055873254, ISSN: 1466-8564, DOI: 10.1016/j.ifset.2013.12.013
- HUANG KAI ET AL: "Optimising separation process of protein and polysaccharide from spent brewer's yeast by ultrafiltration : Optimising separation process of protein and polysaccharide", INTERNATIONAL JOURNAL OF FOOD SCIENCE AND TECHNOLOGY, vol. 47, no. 6, 10 April 2012 (2012-04-10), GB, pages 1259 - 1264, XP055873264, ISSN: 0950-5423, DOI: 10.1111/j.1365-2621.2012.02967.x
- BZDUCHA-WRÓBEL ANNA ET AL: "Evaluation of the Efficiency of Different Disruption Methods on Yeast Cell Wall Preparation for [beta]-Glucan Isolation", MOLECULES, vol. 19, no. 12, 15 December 2014 (2014-12-15), pages 20941 - 20961, XP055873260, DOI: 10.3390/molecules191220941

## Beschreibung

### Technisches Gebiet

Die Erfindung betrifft ein Verfahren zum Aufschliessen von Hefe, insbesondere Bierhefe, zur Isolierung von Proteinen und Sacchariden.

### Stand der Technik

Hefen sind einzellige Pilze, die sich durch Sprossung oder Teilung vermehren und seit jeher von grosser technischer Bedeutung sind. So werden Hefen z.B. bei der Produktion von Bier, Wein, Spirituosen, Lebensmitteln und therapeutischen Substanzen eingesetzt. Grundsätzlich gibt es mehrere Arten von Hefen, wobei die sogenannte Back- oder Bierhefe (Saccharomyces cerevisiae) eine der am häufigsten genutzten Hefen ist. Entsprechend fallen beträchtliche Mengen an Hefe-haltigen und im Besonderen an Bierhefe-haltigen Nebenprodukten an.

Da im Besonderen die Zellwände der Hefen eine Reihe von technisch nutzbaren Biomolekülen, wie beispielsweise Proteine, Glucane, Mannane, Chitine und Lipide enthalten, werden Hefe-haltige Abfälle zwecks Gewinnung dieser Substanzen verarbeitet. Dabei werden die Zellwände der Hefen teilweise oder vollständig aufgeschlossen, um die interessierenden Substanzen freizusetzen.

Die EP 1 990 419 A1 (Tex-a-tec AG) beschreibt beispielsweise ein Verfahren zur Isolierung von Glucan, Protein, Mannan und Lipiden aus Hefe, wobei die Hefe in aufeinanderfolgenden Schritten bei steigenden Temperaturen mit Ultraschall behandelt wird.

Die EP 2 272 876 A1 (Angel Yeast Co., Ltd.) offenbart ein Verfahren zum Extrahieren von Glucan und Mannan aus der Zellwand von Mikroorganismen, wie beispielsweise Hefen8440. Das Verfahren beinhaltet die Schritte: a) Behandeln der Zellen des Mikroorganismus mit einer alkalischen Protease und einer Mannanase; b) Trennen der Mischung aus Schritt a) in eine schwere Phase und eine leichte Phase; c) Trocknen der aus Schritt b) erhaltenen schweren Phase, wobei eine Glucanzubereitung erhalten wird; und d) Trocknen der aus Schritt b) erhaltenen leichten Phase, wobei die Mannan-Zubereitung erhalten wird. Die Glucan- und Mannanzubereitungen sind für therapeutische Anwendungen vorgesehen.

Die WO 2010/070207 A1 (Glykos Finland Oy) beschreibt ein Verfahren zur Herstellung einer immunstimulatorischen Zusammensetzung, die eine Saccharidfraktion umfasst, wobei das Verfahren die Schritte der Hydrolyse von Hefezellen und der Gewinnung einer löslichen Fraktion umfasst. Die so erhaltene Saccharidfraktion kann als Nahrungsmittel- oder Getränkekomponente eingearbeitet werden oder als Pharmazeutikum verwendet werden.

Die bisher bekannten Verfahren zur Isolierung von technisch nutzbaren Biomolekülen aus Hefen vermögen jedoch im Besonderen bei grosstechnischen Ansätzen nicht vollständig zu überzeugen. So ist das Verhältnis von Ausbeute und Aufwand, insbesondere in Bezug auf den Energie- und Zeitbedarf, bei vielen Verfahren unbefriedigend, was eine wirtschaftliche Produktion stark erschwert.

Andere Verfahren sind auf die selektive Gewinnung von einzelnen Komponenten (z.B. Glucan oder Mannan) gerichtet, während die übrigen Komponenten (beispielsweise Proteine) unberücksichtigt bleiben, wie beispielsweise das in EP0950716 (Farmint Group Holding SA) oder das in WO2006/121803 (Sensient Flavors Inc) beschriebene Verfahren. Dies ist im Sinne einer möglichst vollständigen Verwertung von Hefe-haltigen Nebenprodukten nachteilig.

Es besteht daher nach wie vor Bedarf nach verbesserten Lösungen, welche die genannten Nachteile zumindest teilweise oder vollständig überwinden.

### Darstellung der Erfindung

Es ist die Aufgabe der Erfindung, ein verbessertes Verfahren zur Gewinnung von Biomolekülen aus Hefezellen, insbesondere Bierhefezellen, bereitzustellen. Dabei sollen im Besonderen mehrere unterschiedliche Biomoleküle gleichzeitig mit möglichst hohen Ausbeuten und Reinheiten isoliert werden können, besonders bevorzugt Proteine, Glucane und Mannane. Das Verfahren soll zudem eine möglichst effiziente Gewinnung der Biomoleküle bei grosstechnischen Ansätzen erlauben. Wünschenswert ist dabei, dass das Verfahren mit einem möglichst geringen Energie- und Zeitaufwand durchgeführt werden kann.

Die Lösung der Aufgabe ist durch die Merkmale des Anspruchs 1 definiert. Kern der Erfindung ist demnach ein Verfahren zum Aufschliessen von Hefe, insbesondere Bierhefe, zur Isolierung von Proteinen und Sacchariden umfassend die Schritte:
a) Bereitstellen einer Hefesuspension, insbesondere einer Bierhefesuspension;
b) physikalisches Aufschliessen der Hefe in der Hefesuspension;
c) Mikrofiltration der aufgeschlossenen Hefesuspension aus Schritt b);
d) Ultrafiltration des Permeats der Mikrofiltration aus Schritt c) wobei als Retentat der Ultrafiltration eine Phase mit Proteinen als Hauptfeststoffbestandteil abgetrennt wird;
e) Behandeln des Retentats der Mikrofiltration aus Schritt c) mit einer Protease unter basischen Bedingungen und anschliessende Abtrennung einer Phase mit Mannan als Hauptfeststoffbestandteil, insbesondere durch Filtration und/oder Zentrifugieren;
f) Behandeln der in Schritt e) zurückgehaltenen Phase aufeinanderfolgend zuerst bei basischen und danach bei sauren Bedingungen und anschliessende Abtrennung einer Phase mit Glucan als Hauptfeststoffbestandteil, insbesondere durch Filtration und/oder Zentrifugieren.

Es hat sich überraschend gezeigt, dass die erfindungsgemässe Kombination aus physikalischen, chemischen und biochemischen Behandlungsschritten gute Ausbeuten bei verhältnismässig geringem Energie- und Zeitbedarf ermöglicht. Die gilt für alle Produkte, d.h. für Proteine, Glucane und Mannane, wobei Ausbeuten für diese Substanzen von je bis zu ca. 40% bei hoher Reinheit erreicht werden können.

**Des Weiteren** eignet sich das Verfahren für grosstechnische Ansätze. Es können problemlos mehrere zehntausend Tonnen Hefesuspension pro Jahr bzw. mehrere tausend Kilogramm Hefesuspension pro Stunde verarbeitet werden.

"Glucane" sind Oligo- oder Polysaccharide, die aus D-Glucose-Molekülen aufgebaut und durch glycosidische Bindungen miteinander verknüpft sind. Glucane wie sie in Hefezelle vorkommen, sind typischerweise Beta-Glucane, welche eine β-glycosidische Bindung aufweisen, im Besonderen handelt es sich um ß-1,3-Glucan.

"Mannane" sind Polysaccharide, die Polymere des Zuckers Mannose darstellen. Die in Hefen vorkommenden Mannane verfügen typischerweise über ein α(1-6)-verknüpftes Rückgrat sowie α(1-2)- und α(1-3)-verknüpfte Seitenketten, die durchschnittlich etwa zwei Zuckereinheiten lang sind. "Mikrofiltration" meint ein Verfahren zur Filtration durch ein Filtermedium, insbesondere eine Membran, mit einer Porengrösse ≥ 0.1 µm. Dagegen liegt die Porengrösse bei der Ultrafiltration unter 0.1 µm.

Wo nicht anders angegeben, bezieht sich der "Feststoffbestandteil" jeweils auf die Trockenmasse des jeweiligen Bestandteils. Der Ausdruck "Hauptfeststoffbestandteil" bezeichnet denjenigen Bestandteil der Feststoffe mit dem grössten Gewichtsanteil.

Bei der Hefesuspension, welche in Schritt a) bereitgestellt wird, handelt es sich insbesondere um eine bei einer Temperatur von 0 - 15°C, bevorzugt 2 - 12°C, im Besonderen 3 - 7°C oder 5°C gelagerte und/oder eine pasteurisierte Hefesuspension. Dies stellt sicher, dass Suspension möglichst wenig unerwünschte Nebenprodukte enthält.

Grundsätzlich kann es sich bei der in Schritt a) bereitgestellten Hefesuspension aber auch um eine bei einer anderen Temperatur, insbesondere Raumtemperatur, gelagerten und nicht pasteurisierte Hefesuspension handeln.

Bei der Hefesuspension, handelt es sich insbesondere um eine wässrige Hefesuspension. Wasser liegt dabei als kontinuierliche flüssige Phase der Hefesuspension vor.

Die Hefesuspension ist insbesondere eine Bierhefesuspension, welche bevorzugt gebrauchte Bierhefe beinhaltet. Solche Bierhefesuspensionen fallen beispielsweise als Nebenprodukte bei der Bierproduktion an und können im erfindungsgemässen Verfahren direkt eingesetzt werden.

Die Hefesuspension weist bezogen auf das Gesamtgewicht der Hefesuspension bevorzugt einen Feststoffanteil von 5 - 30 Gew.-%, insbesondere 7 - 20 Gew.-%, auf. Dies hat sich für das erfindungsgemässe Verfahren als besonders geeignet herausgestellt. Gegebenenfalls kann der Feststoffanteil der Hefesuspension mit Flüssigkeit, insbesondere Wasser, im gewünschten Bereich eingestellt werden.

Die Hefesuspension weist in Schritt a) insbesondere eine Temperatur von 0 - 15°C, bevorzugt 2 - 12°C, im Besonderen 3 - 7°C oder 5°C, auf. Dies stellt sicher, dass sich die Zusammensetzung der Suspension nicht weiter verändert, z.B. durch aktive Hefezellen, bzw. die Bildung von unerwünschten Nebenprodukten reduziert wird.

Weiter bevorzugt erfolgt auch Schritt b), und optional auch die Schritte c) und d), bei einer Temperatur von 0 - 15°C, bevorzugt 2 - 12°C, im Besonderen 3 - 7°C oder 5°C. Dies reduziert die Bildung von unerwünschten Nebenprodukten weiter.

Grundsätzlich kann die Hefesuspension in den Schritten a), b), c) und/oder d) aber auch eine andere Temperatur aufweisen, beispielsweise Raumtemperatur.

Gemäss einer vorteilhaften Ausführungsform wird die bereitgestellte Hefesuspension vor Schritt b) einem Waschprozess unterzogen, bei welchem wenigstens ein Teil der flüssigen Phase der Hefesuspension, im Besonderen wenigstens 50 Gew.-%, im Speziellen wenigstens 60 Gew.-%, der flüssigen Phase der Hefesuspension, durch eine weitere Flüssigkeit bzw. eine Austauschflüssigkeit, insbesondere Wasser, ausgetauscht wird. Der Waschprozess erfolgt bevorzugt in einer Zentrifuge oder einem Trommelfilter.

Durch den Waschprozess lässt sich bei Bedarf der Anteil von in der Flüssigkeit gelösten und unerwünschten Bestandteilen reduzieren, womit sich die Reinheit und Qualität der zu isolierenden Substanzen verbessern lässt. Ein Waschprozess ist aber optional und kann auch weggelassen werden.

Das physikalische Aufschliessen in Schritt b) erfolgt insbesondere durch elektrische Felder, in einem Homogenisator oder einer Mahlkörpermühle, insbesondere in einer Kugelmühle.

Der Homogenisator ist insbesondere ein Hochdruckhomogenisator, welcher im Besonderen mit einem Druck von mehr als 1'000 bar, im Speziellen mehr als 1'200 bar betrieben wird.

Bei einer Mahlkörpermühle werden in einem Prozessraum Mahlgut und frei bewegliche Mahlkörper, insbesondere Kugeln, umgewälzt. Dabei kommt es zu Stössen zwischen den Mahlkörpern, den Wänden des Prozessraums und dem Mahlgut. Die Zerkleinerung des Mahlguts erfolgt dabei vornehmlich durch Zertrümmerung.

Das Aufschliessen mit einer Mahlkörpermühle, im Besonderen einer Kugelmühle, hat sich vorliegend als besonders vorteilhaft erwiesen, da die zu isolierenden Proteine schonend, aber dennoch effektiv freigesetzt werden können. Dies gilt im Besonderen für Bierhefesuspensionen. Für spezielle Anwendungen können aber auch andere Verfahren zum physikalischen Aufschliessen eingesetzt werden.

Das physikalische Aufschliessen durch elektrische Felder in Schritt b) kann insbesondere durch gepulste elektrische Felder (im Englischen auch als PEF bzw. "Pulsed Electric Fields" bezeichnet) erfolgen. Diese Aufschlusstechnik ist dem Fachmann in anderen Zusammenhängen an sich bekannt.

Bei der Behandlung durch gepulste elektrische Felder wird die Hefesuspension insbesondere zwischen zwei Elektroden platziert und mit gepulsten elektrischen Feldern behandelt. Die gepulsten elektrischen Felder zeichnen sich insbesondere durch hohe Feldstärken und eine kurze Dauer aus. Dabei findet eine reversible oder irreversible Porenbildung in den Zellmembranen statt, wodurch intrazelluläre Bestandteile freigesetzt werden und/oder extrazelluläre Substanzen in die Zellen eindringen können.

Vorliegend haben sich insbesondere die folgenden Parameter als geeignet herausgestellt, um das Aufschliessen der Hefe in der Hefesuspension zu kontrollieren: die elektrische Feldstärke [kV/cm], die Eingangstemperatur der Hefesuspension [°C] und der spezifische Energieeintrag [kJ/L]. Als Energieeintrag wird die für das Verfahren benötigte Energie in Kilojoule pro Liter Hefesuspension verstanden. Ausserdem kann der Energieeintrag über die Frequenz der Pulse [Hz] gesteuert werden, welche wiederum vom Durchfluss abhängt und bei bekannten Anlagen automatisch kontrolliert wird.

Bevorzugt beträgt die elektrische Feldstärke 1 - 30 kV/cm, insbesondere 5 - 24 kV/cm.

Der spezifische Energieeintrag beträgt vorzugsweise 1 - 180 kJ/L, insbesondere 3 - 120 kJ/L.

Die Eingangstemperatur der Hefesuspension beträgt vorzugsweise 0 - 15°C, bevorzugt 2 - 12°C, im Besonderen 3 - 7°C oder 5°C.

Mit derartigen Parametern kann die Hefe in der Hefesuspension effektiv aufgeschlossen werden.

Anlagen zur Durchführung eines physikalischen Zellaufschlusses mittels gepulster elektrischer Felder sind kommerziell bei verschiedenen Anbietern erhältlich, wie z.B. die Anlage PEF Pilot Dual (Elea-Technology; Deutschland).

Der Behandlung durch gepulste elektrische Felder folgt vorzugsweise eine mechanische Nachbehandlung. Diese erfolgt somit bevorzugt zwischen Schritt b) und c).

Die mechanische Nachbehandlung kann insbesondere mittels eines Mischers erfolgen, bevorzugt mit hohen Scherkräften. Insbesondere geeignet ist ein Rotor-Stator-Mischer, im Besonderen ein Inline Rotor-Stator-Mischer. Bei einem Inline Rotor-Stator-Mischer sind Rotor und Stator typischerweise in einem Gehäuse mit einem Einlass an einem Ende und einem Auslass am anderen Ende enthalten. Die zu behandelnde Hefesuspension kann dabei in einem kontinuierlichen Strom durch den Mischer gezogen werden.

Die Schergeschwindigkeit bei der mechanischen Nachbehandlung mit einem Mischer wird insbesondere derart gewählt, dass die Hefezellen weiter aufgeschlossen werden. Dadurch können die nach Anwendung der gepulsten elektrischen Felder allenfalls noch nicht vollständig freigesetzten intrazellulären Bestandteile (Proteine) freigesetzt werden.

Durch die hohen Scherkräfte werden die bereits geöffneten Hefezellen zusätzlich "ausgequetscht" und intrazelluläre Bestandteile, insbesondere die Proteine, können aus den Zellen entweichen.

Die Mikrofiltration in Schritt c) erfolgt vorzugsweise mit einem Filtermedium mit einer Porengrösse von 0.1 - 0.5 µm, bevorzugt 0.1 - 0.2 µm, insbesondere 0.1 µm. Dies hat sich als besonders bevorzugt erwiesen, um nach dem physikalischen Aufschliessen bezogen auf den Feststoffgehalt eine relative Anreicherung der zu isolierenden Proteine im Permeat und zugleich eine Anreicherung von Biomassenreste, z.B. Zellfragmente und Fette, im Retentat zu erreichen.

Im Besonderen wird die Mikrofiltration in Schritt c) als Diafiltration durchgeführt, wobei vorlagenseitig bevorzugt fortlaufend eine Austauschflüssigkeit, insbesondere Wasser, zugeführt wird. Anders ausgedrückt wird bei der Diafiltration bevorzugt die flüssige Phase der aufgeschlossenen Hefesuspension, welche als Permeat abgeführt wird, vorlagenseitig zumindest teilweise, insbesondere vollständig, fortlaufend durch eine Austauschflüssigkeit ersetzt, insbesondere durch Wasser.

Die aufgeschlossene Hefesuspension wird insbesondere vom einem Vorlagenbehälter aus zirkuliert. Durch das Regulieren des Flusses nach dem Filtermedium kann ein Transmembrandruck eingestellt werden, der als treibende Kraft in der Diafiltration wirkt.

Die Diafiltration wird bevorzugt in einem geschlossenen Kreislauf betrieben, so dass das Abführen des Permeates zum Nachfliessen des Austauschlösungsmittels führt, insbesondere so, dass das zirkulierende Volumen während der Diafiltration konstant bleibt.

Konkret wird die Mikrofiltration in Schritt c) bevorzugt derart durchgeführt, dass ein Anteil an Proteinen im Permeat der Mikrofiltration aus Schritt c), bezogen auf das Gesamtgewicht der Feststoffe im Permeat, bevorzugt wenigstens 50 Gew.-%, weiter bevorzugt 50 - 80 Gew.-%, insbesondere 60 - 70 Gew.-%, beträgt.

Ein Gesamtfeststoffanteil im Permeat der Mikrofiltration in Schritt c) beträgt vorzugsweise 1 - 10 Gew.-%, insbesondere 3 - 7 Gew.-%, bezogen auf das Gesamtgewicht des Permeats.

Weiter bevorzugt wird die Mikrofiltration in Schritt c) derart durchgeführt, dass ein Anteil an Proteinen im Retentat der Mikrofiltration aus Schritt c) bezogen auf das Gesamtgewicht der Feststoffe im Retentat weniger als 50 Gew.-%, bevorzugt weniger als 40 Gew.-%, insbesondere weniger als 35 Gew.-%, beträgt.

Ein Gesamtfeststoffanteil im Retentat der Mikrofiltration in Schritt c) beträgt vorzugsweise 20 - 50 Gew.-%, insbesondere 25 - 30 Gew.-%, bezogen auf das Gesamtgewicht des Retentats.

Gemäss einer weiteren möglichen Ausführungsform wird die aufgeschlossene Hefesuspension vor dem Mikrofiltrationsschritt c) mit einem Oxidationsmittel, insbesondere Ozon (O₃) und/oder Wasserstoffperoxid (H₂O₂), behandelt. Damit können im Besonderen chemische Reaktionen, welche zu unerwünschten Nebenprodukten und/oder Geschmacksveränderungen führen, unterbunden werden. Die Behandlung mit einem Oxidationsmittel ist aber optional.

Weiter kann die aufgeschlossene Hefesuspension vor dem Mikrofiltrationsschritt c) enzymatisch behandelt werden. Dadurch können bei Bedarf die Effizienz und Ausbeute des Verfahrens erhöht werden. Eine enzymatische Behandlung vor dem Mikrofiltrationsschritt c) ist aber optional.

Die Ultrafiltration in Schritt d) erfolgt vorzugsweise mit einem Filtermedium mit einer Porengrösse von kleiner als 90 nm und/oder einer Ausschlussgrenze (Nominal Molecular Weight Cut-Off) von 1 - 100 kDa, im Speziellen 5 - 100 kDa, bevorzugt 10 - 50 kDa, insbesondere 15 - 25 kDa, im Speziellen 20 kDa. Die Ausschlussgrenze ist definiert als die minimale Molekülmasse von Molekülen, welche durch das Filtermedium, insbesondere die Membran, zu 90% zurückgehalten werden.

Im Besonderen wird die Ultrafiltration in Schritt d) als Diafiltration durchgeführt, wobei vorlagenseitig bevorzugt fortlaufend eine Austauschflüssigkeit, insbesondere Wasser, zugeführt wird. Anders ausgedrückt wird bei der Diafiltration in Schritt d) bevorzugt die flüssige Phase des Permeats der Mikrofiltration aus Schritt c), welche in der Diafiltration als Permeat abgeführt wird, vorlagenseitig zumindest teilweise, insbesondere vollständig, fortlaufend durch eine Austauschflüssigkeit ersetzt, insbesondere durch Wasser.

Das Permeat der Mikrofiltration aus Schritt c), welches die Vorlage in Schritt d) bildet, wird insbesondere von einem Vorlagenbehälter aus zirkuliert. Durch das Regulieren des Flusses nach dem Filtermedium kann wiederum ein Transmembrandruck eingestellt werden, der als treibende Kraft der Diafiltration in Schritt d) wirkt.

Die Diafiltration in Schritt d) wird bevorzugt in einem geschlossenen Kreislauf betrieben, so dass das Abführen des Permeats zum Nachfliessen der Austauschflüssigkeit führt, insbesondere so, dass das zirkulierende Volumen während der Diafiltration konstant bleibt.

Durch die Ultrafiltration und die Diafiltration in Schritt d) kann der relative Feststoffanteil des Proteins im Retentat gezielt erhöht werden.

Bevorzugt wird die Ultrafiltration in Schritt d) derart durchgeführt, dass ein Anteil der Proteine in der in Schritt d) abgetrennten Phase mit Proteinen als Hauptfeststoffbestandteil bezogen auf das Gewicht der Feststoffe wenigstens 75 Gew.-%, insbesondere wenigstens 85 Gew.-%, bevorzugt wenigstens 90 Gew.-%, beträgt.

Ein Gesamtfeststoffanteil in der in Schritt d) abgetrennten Phase beträgt vorzugsweise 0.5 - 7 Gew.-%, insbesondere 1 - 3 Gew.-%, bezogen auf das Gesamtgewicht der in Schritt d) abgetrennten Phase.

Im Besonderen werden sowohl die Mikrofiltration in Schritt c) und als auch die Ultrafiltration in Schritt d) als Diafiltration durchgeführt.

Besonders bevorzugt wird die in Schritt d) abgetrennte Phase mit Proteinen als Hauptfeststoffbestandteil nach Schritt d) getrocknet, insbesondere sprühgetrocknet. Dadurch ist das Protein als pulverförmiges Produkt mit hoher Reinheit erhältlich. Entsprechende Vorrichtungen und Verfahren zur Sprühtrocknung sind dem Fachmann an sich bekannt.

Besonders bevorzugt wird vor der Sprühtrocknung der Feststoffgehalt, im Besonderen der Proteingehalt, aufkonzentriert, insbesondere auf einen Feststoffgehalt von wenigstens 50 Gew.-%, im Besonderen wenigstens 60 Gew.-%, bevorzugt wenigstens 70 Gew.-%. Die Aufkonzentrierung erflogt insbesondere in einem Verdampfer, welcher die flüssige Phase teilweise verdampft. Entsprechende Vorrichtung und Verfahren zum Aufkonzentrieren sind dem Fachmann an sich bekannt.

Optional kann die in Schritt d) abgetrennte Phase mit Proteinen als Hauptfeststoffbestandteil nach Schritt d) mit einem Oxidationsmittel, insbesondere Ozon und/oder Wasserstoffperoxid, behandelt und/oder mit einem Adsorbermaterial, z.B. Aktivkohle, kontaktiert werden. Dadurch kann insbesondere die Produktqualität der Proteine verbessert werden. Beispielsweise lassen sich unerwünschte Verfärbungen und/oder Geschmacksstoffe chemisch zersetzen und/oder entfernen.

Falls eine Sprühtrocknung durchgeführt wird, erfolgt die Behandlung mit dem Oxidationsmittel und/oder das Kontaktieren mit dem Adsorbermaterial bevorzugt vor der Sprühtrocknung und, sofern ein solches erfolgt, vor dem Aufkonzentrieren.

Eine Behandlung mit einem Oxidationsmittel wird besonders bevorzugt mit einem Membrankontaktor durchgeführt. Bei einem Membrankontaktor werden die im Stoffaustausch befindlichen Fluide getrennt von einer porösen Membran aneinander vorbeigeführt. Im vorliegenden Fall handelt es sich bei den Fluiden um die in Schritt d) abgetrennte Phase mit Proteinen als Hauptfeststoffbestandteil und das Oxidationsmittel. Das Oxidationsmittel gelangt im Membrankontaktor durch die Membran hindurch in die in Schritt d) abgetrennte Phase mit Proteinen als Hauptfeststoffbestandteil. Der Einsatz eines Membrankontaktors hat sich im Vergleich mit anderen Kontaktoren vorliegend als besonders vorteilhaft und effizient erwiesen. Dies im Besonderen da der Druckverlust und Energiebedarf im Membrankontaktor relativ gering ist, das Oxidationsmittel seine Wirkung aber dennoch sehr effektiv entfalten kann.

In Schritt e) wird eine Protease eingesetzt. Proteasen sind Enzyme, die Proteine hydrolytisch spalten können. Proteasen werden auch proteolytische Enzyme genannt.

Bevorzugt wird in Schritt e) die Protease mit einem Anteil von 0.0001-10 Gew.-%, insbesondere 0.001-5 Gew.-%, im Speziellen 0.01-1 Gew.-% oder 0.05-0.5 Gew.-%, bezogen auf den Feststoffgehalt des Retentats der Mikrofiltration aus Schritt c), verwendet.

Bei der Protease handelt es sich bevorzugt um eine alkalische Protease, im Besonderen um eine Serinprotease, besonders bevorzugt um ein Subtilisin.

Serinproteasen sind eine Unterfamilie der Proteasen, welche im aktiven Zentrum über die Aminosäure Serin verfügen.

Proteasen vom Subtilisin-Typ (Subtilasen, Subtilopeptidasen, EC 3.4.21.62) werden aufgrund der katalytisch wirksamen Aminosäuren den Serinproteasen zugerechnet. Sie werden natürlicherweise von Mikroorganismen, insbesondere von Bacillus-Spezies gebildet und sekretiert. Sie wirken als unspezifische Endopeptidasen, das heisst sie hydrolysieren beliebige Säureamidbindungen, die im Inneren von Peptiden oder Proteinen liegen. Ihr pH-Optimum liegt meist im deutlich alkalischen Bereich.

Proteasen und insbesondere die genannten spezifischen Proteasen haben sich im vorliegenden Verfahren als äusserst vorteilhaft herausgestellt. Im Besonderen kann durch die Protease selektiv Mannan freigesetzt werden, welches sich in Form einer flüssigen Phase mit Mannan als Hauptfeststoffbestandteil abtrennen lässt.

Im Besonderen wird das Verfahren ohne Zugabe von Mannanase oder in Abwesenheit von Mannanase durchgeführt. Mannanase ist ein Enzym, das Mannane abbaut.

Die Behandlung in Schritt e) erfolgt vorzugsweise bei einem pH-Wert von 7.5 - 13, bevorzugt 8 - 11, im Besonderen 8.5 - 10.5, und/oder bei einer Temperatur von 40 - 80°C, bevorzugt 50 - 70°C, insbesondere 55 - 65°C. Der pH-Wert wird bevorzugt auf die verwendete Protease abgestimmt.

Insbesondere wird in Schritt e) der pH-Wert durch Zugabe einer Base, bevorzugt einer Base mit einem pK_{B}-Wert von weniger als 4.75, im Besonderen weniger als 1, eingestellt. Der pK_{B}-Wert steht für den negativen dekadischen Logarithmus der Basenkonstante K_{B}. Besonders bevorzugt handelt es sich um eine anorganische Base, im Speziellen NaOH. Derartige Basen haben sich als besonders geeignet herausgestellt.

Die Base wird vorzugsweise in verdünnter Form, z.B. mit einem Anteil von 30 - 60 Gew.-% in Wasser, eingesetzt.

Die Behandlung in Schritt e) dauert im Besonderen 10 min bis 18 Stunden, insbesondere 3 - 12 Stunden. Dadurch kann die Ausbeute optimiert werden.

In einer weiteren Ausführungsform lässt sich in Schritt e) eine pastöse Fraktion mit einer geringen Menge an Flüssigkeit verarbeiten. Dadurch kann je nach Zusammensetzung der Hefesuspension und Reaktionsführung der enzymatische Schritt hinsichtlich Ausbeute optimiert werden.

In Schritt f) wird die zurückgehaltene Phase vorzugsweise in einem ersten Zeitintervall, bevorzugt für 1 - 5 Stunden, bei einem pH-Wert von 7.5 - 13, bevorzugt 8 - 11, im Besonderen 8.5 - 10.5, und/oder bei einer Temperatur von 40 - 95°C, bevorzugt 65 - 85°C, insbesondere 75 - 85°C, behandelt. Anschliessend wird die zurückgehaltene Phase vorzugsweise in einem zweiten Zeitintervall, bevorzugt für 0.5 - 2 Stunden, bei einem pH-Wert von 2 - 6.5, bevorzugt 3 - 5, im Besonderen 3.5 - 4.5, und/oder bei einer Temperatur von 50 - 95°C, bevorzugt 80 - 95°C, insbesondere 80 - 90°C, behandelt.

Mit der in Schritt f) genannten "in Schritt e) zurückgehaltenen Phase" ist insbesondere diejenige Phase gemeint, die nach Abtrennung der Phase mit Mannan als Hauptfeststoffbestandteil unter Schritt e) zurückbleibt. Insbesondere entspricht die "in Schritt e) zurückgehaltenen Phase" somit dem Retentat aus Schritt c), von welchem die Phase mit Mannan als Hauptfeststoffbestandteil abgetrennt wurde.

Erfolgt eine Abtrennung der Phase mit Mannan als Hauptfeststoffbestandteil in Schritt e) beispielsweise durch Filtration, ist die "in Schritt e) zurückgehaltenen Phase" auf welche in Schritt f) Bezug genommen wird insbesondere das Retentat aus Schritt e). In diesem Fall bildet die Phase mit Mannan als Hauptfeststoffbestandteil in Schritt e) das Permeat, welches abgetrennt wird.

Besonders bevorzugt wird in Schritt f) der pH-Wert im ersten Zeitintervall derart gewählt, dass er dem bei der Behandlung in Schritt e) vorliegenden pH-Wert entspricht.

Weiter ist es bevorzugt, wenn in Schritt f) die Temperatur im ersten Zeitinterwall höher ist als die bei der Behandlung in Schritt e) vorliegende Temperatur.

Vorzugsweise ist in Schritt f) die Temperatur im ersten Zeitinterwall geringer ist als im zweiten Zeitintervall und/oder das erste ist Zeitintervall länger ist als das zweite Zeitintervall.

In Schritt f) wird, sofern erforderlich, der pH-Wert durch Zugabe einer Base, bevorzugt NaOH, und/oder durch Zugabe einer Säure, bevorzugt H₂SO₄, eingestellt.

Die Base ist dabei bevorzugt eine Base wie sie vorstehend bei Schritt e) beschrieben ist. Besonders bevorzugt handelt es sich bei der Base in Schritt f) um die gleiche Base wie in Schritt e).

Bei der Säure handelt es sich insbesondere um eine Säure mit einem pK_{S}-Wert von weniger als 4.75, im Besonderen weniger als 1. Der pK_{S}-Wert steht für den negativen dekadischen Logarithmus der Säurenkonstante K_{S}. Besonders bevorzugt handelt es sich um eine Mineralsäure, insbesondere H₂SO₄.

Die Säure wird vorzugsweise in verdünnter Form, z.B. mit einem Anteil von 30 - 60 Gew.-% in Wasser, eingesetzt.

Diese vorgenannten Massnahmen, insbesondere in Kombination, haben sich in Bezug auf die Effizienz des Verfahrens als besonders vorteilhaft herausgestellt.

Die Abtrennung der Phase mit Mannan als Hauptfeststoffbestandteil in Schritt e) erfolgt vorzugsweise durch Filtration, insbesondere eine kombinierte Mikrofiltration und Ultrafiltration. Ebenso erfolgt die Abtrennung der Phase mit Glucan als Hauptfeststoffbestandteil in Schritt f) bevorzugt durch Filtration, insbesondere eine kombinierte Mikrofiltration und Ultrafiltration.

Die nach Abtrennung der Phase mit Mannan unter Schritt e) zurückbleibende Phase, wird in Schritt f) insbesondere als die "zurückgehaltene Phase" weiterbehandelt.

Die Mikrofiltration wird dabei jeweils vorzugsweise mit einem Filtermedium mit einer Porengrösse von 0.1 - 0.5 µm, insbesondere 0.1 - 0.2 µm, bevorzugt 0.1 µm durchgeführt. Die Ultrafiltration wird bevorzugt jeweils mit einem Filtermedium mit einer Porengrösse von kleiner als 90 nm und/oder einer Ausschlussgrenze (Nominal Molecular Weight Cut-Off) von 1 - 100 kDa, im Speziellen 5 - 100 kDa, bevorzugt 10 - 50 kDa, insbesondere 15 - 25 kDa, im Speziellen 20 kDa, durchgeführt.

Die Mikrofiltration in Schritt e) und/oder die Ultrafiltration in Schritt f) werden vorzugsweise als Diafiltration durchgeführt, insbesondere in der gleichen Weise wie dies bei den Schritten c) und d) beschrieben wurde.

Dabei wird in Schritt e) insbesondere die Phase mit Mannan als Hauptfeststoffbestandteil in Form des Permeats der Mikrofiltration abgeführt, der Ultrafiltration zugeführt und der relative Anteil des Mannans im Retentat der Ultrafiltration erhöht.

In Schritt f) wird insbesondere die Phase mit Glucan als Hauptfeststoffbestandteil in Form des Permeats der Mikrofiltration abgeführt, der Ultrafiltration zugeführt und der relative Anteil des Glucans im Retentat der Ultrafiltration erhöht.

Bei der Diafiltration in den Schritten e) und/oder f) werden die Flüssigkeiten insbesondere vom Reaktionsbehälter, in welchem die Schritte e) und/oder f) stattfinden, zirkuliert, wobei vorlagenseitig bevorzugt fortlaufend Austauschflüssigkeit zugeführt wird.

Gemäss einer weiteren vorteilhaften Ausführungsform erfolgt das Abtrennen in den Schritten e) und/oder f) durch einen Trommelfilter und/oder eine Zentrifuge. Dies insbesondere in Schritt f).

In einer besonderen Ausführungsform erfolgt demnach die Abtrennung in Schritt e) durch eine kombinierte Mikrofiltration und Ultrafiltration, wie dies vorstehend beschrieben ist, und die Abtrennung in Schritt f) erfolgt durch einen Trommelfilter und/oder eine Zentrifuge, insbesondere eine Zentrifuge.

Bevorzugt wird die in Schritt e) abgetrennte Phase mit Mannan als Hauptfeststoffbestandteil nach Schritt e) getrocknet, insbesondere sprühgetrocknet, und/oder die in Schritt f) abgetrennte Phase mit Glucan als Hauptfeststoffbestandteil wird nach Schritt f) getrocknet, insbesondere sprühgetrocknet. Dadurch sind sowohl Mannan als auch Glucan als pulverförmige Produkte mit hoher Reinheit erhältlich.

Vorzugsweise wird vor der Sprühtrocknung der Feststoffgehalt, im Besonderen der Gehalt an Mannan und/oder Glucan, aufkonzentriert, insbesondere mit einem Verdampfer.

Optional kann die in Schritt e) abgetrennte Phase mit Mannan als Hauptfeststoffbestandteil nach Schritt e) mit einem Oxidationsmittel, insbesondere Ozon und/oder Wasserstoffperoxid, behandelt und/oder mit einem Adsorbermaterial, z.B. Aktivkohle, kontaktiert werden.

Ebenso kann optional die in Schritt f) abgetrennte Phase mit Glucan als Hauptfeststoffbestandteilmit nach Schritt f) mit einem Oxidationsmittel, insbesondere Ozon und/oder Wasserstoffperoxid, behandelt und/oder mit einem Adsorbermaterial, z.B. Aktivkohle, kontaktiert werden.

Damit kann wie bei der Proteinabtrennung die Produktqualität von Mannan und/oder Glucan verbessert werden.

Falls nach den Schritten e) und/oder f) eine Sprühtrocknung durchgeführt wird, erfolgt die Behandlung mit dem Oxidationsmittel und/oder das Kontaktieren mit dem Adsorbermaterial bevorzugt vor der Sprühtrocknung und, sofern ein solches erfolgt, vor dem Aufkonzentrieren.

Die Behandlung mit einem Oxidationsmittel wird besonders bevorzugt mit einem Membrankontaktor durchgeführt. Diesbezügliche Vorteile wurden vorstehend im Zusammenhang mit dem Proteinstrom beschrieben.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

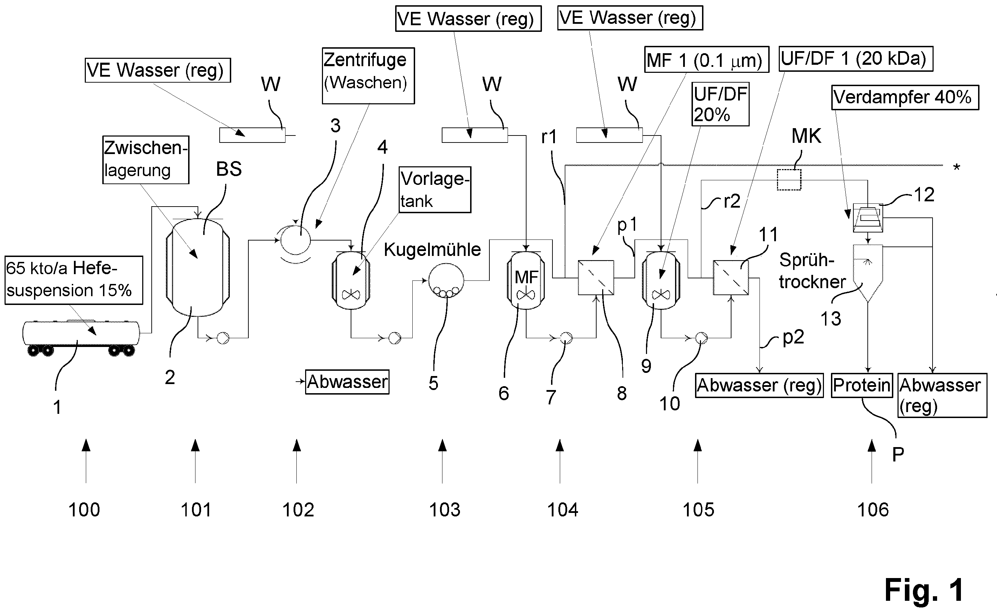

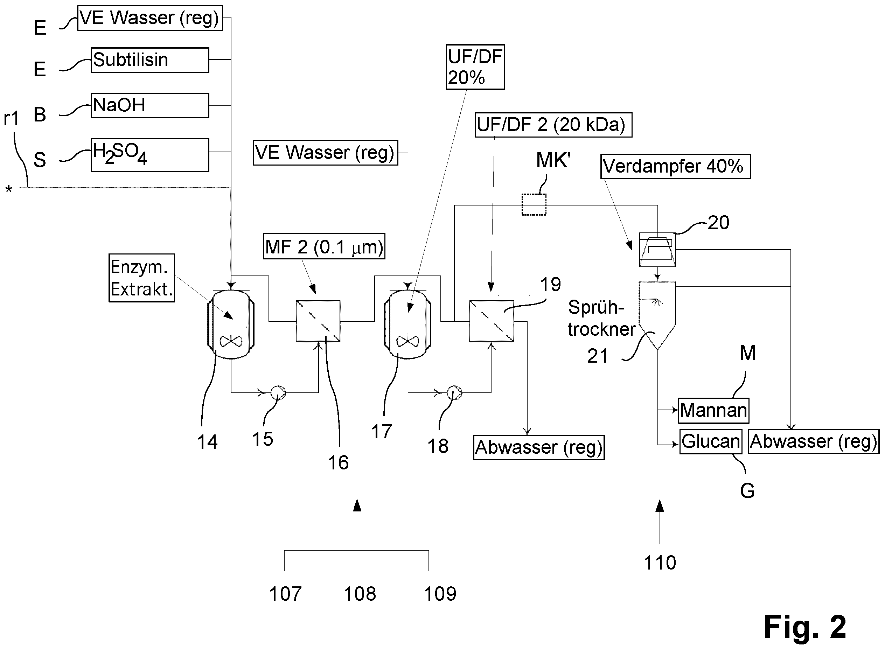

Die zur Erläuterung des Ausführungsbeispiels verwendeten Fig. 1 und 2 zeigen das Verfahrensfliessbild eines erfindungsgemässen Verfahrens zum Aufschliessen von Bierhefe zwecks Isolierung von Proteinen (P), Mannan (M) und Glucan (G).

Grundsätzlich sind in den Figuren gleiche Teile mit gleichen Bezugszeichen versehen.

### Wege zur Ausführung der Erfindung

Die Fig. 1 und 2 zeigen das Verfahrensfliessbild eines erfindungsgemässen Verfahrens zum Aufschliessen von Bierhefe zwecks Isolierung von Proteinen (P), Mannan (M) und Glucan (G).

In einem ersten Schritt 100 wird eine gekühlte wässrige Bierhefesuspension BS (Temperatur = 5°C; Feststoffkonzentration = 15 Gew.-%) mit einem Tanklaster 1 angeliefert, um im darauffolgenden Schritt 101 in einem Lagertank 2 bei 5°C zwischengelagert zu werden.

Die Bierhefesuspension BS wird sodann in einer Zentrifuge 3 einem Waschprozess 102 unterzogen. Dabei wird die flüssige Phase der Bierhefesuspension BS durch Wasser W ausgetauscht und einem Vorlagetank 4 zugeführt.

Aus dem Vorlagetank 4 wird die gewaschene Bierhefesuspension sodann in eine Kugelmühle 5 geleitet, wo die Bierhefezellen in der Suspension im nächsten Prozessschritt 103 physikalisch aufgeschlossen werden. Die aufgeschlossene Bierhefesuspension wird daraufhin einem Mikrofiltrationsschritt 104 unterzogen. Die Mikrofiltration wird als Diafiltration mit einem Filtermedium mit einer mit Porengrösse von 0.1 µm durchgeführt. Die aufgeschlossene Bierhefesuspension wird dabei von einem Vorlagenbehälter 6 mit einer Pumpe 7 über den Mikrofilter 8 zirkuliert, wobei vorlagenseitig fortlaufend Wasser W als Austauschflüssigkeit zugeführt wird. Der Transmembrandruck, welcher sich durch den Permeatabfluss regulieren lässt, beträgt ca. 1.5 bar. Ein Anteil an Proteinen im Permeat p1 der Mikrofiltration, bezogen auf das Gesamtgewicht der Feststoffe im Permeat p1, beträgt beispielsweise 60 - 70 Gew.-%.

Das Permeat p1 des Mikrofiltrationsschritts 104 wird sodann einem Ultrafiltrationsschritt 105 unterzogen. Die Ultrafiltration wird als Diafiltration mit einem Filtermedium mit einer Ausschlussgrenze (Nominal Molecular Weight Cut-Off) von 20 kDa durchgeführt. Das Permeat p1 wird dabei von einem weiteren Vorlagenbehälter 9 mit einer Pumpe 10 über den Ultrafilter 11 zirkuliert, wobei vorlagenseitig fortlaufend Wasser W als Austauschflüssigkeit zugeführt wird. Der Transmembrandruck, welcher sich durch den Permeatabfluss regulieren lässt, beträgt ca. 2 bar. Durch die Ultrafiltrationsschritt 105 wird der relative Feststoffanteil des Proteins im Retentat r2 erhöht, so dass ein Feststoffanteil der Proteine bezogen auf den Gesamtfeststoffgehalt im verbleibenden Retentat r2 ca. 90 Gew.-%, beträgt.

Das Permeat p2 des Ultrafiltrationsschritts 105 wird als Abwasser abgeführt (dieses kann bei Bedarf wiederaufbereitet werden).

Das Retentat r2 des Ultrafiltrationsschritts 105 mit Proteinen als Hauptfeststoffbestandteil wird einem Trocknungsprozess 106 unterzogen, wobei das Retentat r2 nach dem Durchlaufen eines Verdampfers 12 einer Sprühtrocknungsvorrichtung 13 zugeführt wird. Daraus resultiert das Protein als pulverförmiges Produkt P.

Die Ausbeute an Protein über den Gesamtprozess beträgt ca. 38 Gew.-% bezogen auf den Gehalt an verfügbaren Proteinen in der Bierhefesuspension BS.

Das Retentat r1 des Mikrofiltrationsprozesses 104 wird schrittweise enzymatisch und chemisch weiterbehandelt, was in Fig. 2 dargestellt ist. Fig. 2 schliesst mit der mit einem Stern (*) bezeichneten Stelle an die mit einem Stern bezeichnete Stelle in Fig. 1 an.

In einem proteolytischen Verdauungsschritt 107 wird das Retentat r1 in einem Reaktor 14 beispielsweise während 8 Stunden mit 0.2 Gew.-% (bezogen auf den Feststoffgehalt des Retentats r1) einer Protease E in Form von Subtilisin behandelt. Die Behandlung erfolgt bei einem pH-Wert von ca. 9.5, wobei der pH-Wert durch Zugabe von Base B in Form einer 45%-igen wässrigen Lösung von NaOH eingestellt und während der Behandlung mit Protease konstant gehalten wird.

Durch den proteolytischen Verdau wird selektiv Mannan freigesetzt, welches anschliessend über eine Mikrofiltration und nachgeschaltete Ultrafiltration in Form einer wässrigen Phase mit Mannan als Hauptfeststoffbestandteil abgetrennt wird. Die Mikrofiltration als auch die Ultrafiltration werden als Diafiltrationen durchgeführt. Während der Mikrofiltration wird die zu filtrierende Flüssigkeit mit einer Pumpe 15 aus dem Reaktor 14 über den Mikrofiltrationsfilter 16 (Porengrösse: 0.1 µm) zirkuliert, wobei vorlagenseitig fortlaufend Austauschflüssigkeit zugeführt wird. Das Permeat der Mikrofiltration wird danach dem Vorlagenbehälter 17 der Ultrafiltrationsstufe zugeführt aus welcher die Flüssigkeit mit einer Pumpe 18 über den Ultrafilter 19 (Ausschlussgrenze: 20 kDa) zirkuliert wird. Durch die Ultrafiltration wird der relative Feststoffanteil des Mannans im Retentat der Ultrafiltrationsstufe erhöht, so dass ein Feststoffanteil an Mannan bezogen auf den Gesamtfeststoffgehalt im verbleibenden Retentat ca. 65 Gew.-% beträgt. Das Permeat der Ultrafiltrationsstufe wird als Abwasser abgeführt (dieses kann bei Bedarf wiederaufbereitet werden).

Das Retentat der Ultrafiltrationsstufe mit Mannan als Hauptfeststoffbestandteil wird einem Trocknungsprozess 110 unterzogen, wobei das Retentat nach dem Durchlaufen eines Verdampfers 20 einer Sprühtrocknungsvorrichtung 21 zugeführt wird. Daraus resultiert das Mannan als pulverförmiges Produkt M.

Die Ausbeute an Mannan über den Gesamtprozess beträgt ca. 39 Gew.-% bezogen auf den Gehalt an verfügbaren Mannan in der Bierhefesuspension BS.

Sodann wird die im Reaktor 14 zurückgehaltene Phase auf eine Temperatur von ca. 80°C erhitzt und in einem ersten Zeitintervall von ca. 3 Stunden einer basischen Extraktion 108 bei einem pH-Wert von 9.5 unterzogen. Der pH-Wert wird durch kontrollierte Zugabe von verdünnter NaOH konstant gehalten.

Anschliessend wird die Temperatur nochmals erhöht auf ca. 85°C und der pH-Wert durch Zugabe von 50%-iger H₂SO₄ in Wasser auf einen Wert von 4 abgesenkt. Daraufhin wird die im Reaktor zurückgehaltene Phase in einem Zeitintervall von 1 Stunde einer sauren Extraktion 109 unterzogen.

Nach erfolgter basischer und saurer Extraktion wird analog zum Mannan eine flüssige Phase mit Glucan als Hauptbestandteil über die beiden Filterstufen (Mikrofilter 16 und Ultrafilter 18) durch Diafiltration abgetrennt. Dadurch wird ein Feststoffanteil an Glucan bezogen auf den Gesamtfeststoffgehalt im Retentat der Diafiltrationsstufe von ca. 72 Gew.-% erreicht.

Das Retentat der Ultrafiltrationsstufe mit Glucan als Hauptfeststoffbestandteil wird ebenfalls einem Trocknungsprozess 110 unterzogen, wobei das Retentat nach dem Durchlaufen des Verdampfers 20 der Sprühtrocknungsvorrichtung 21 zugeführt wird. Daraus resultiert das Glucan als pulverförmiges Produkt G.

Die Ausbeute an Glucan über den Gesamtprozess beträgt ca. 42 Gew.-% bezogen auf den Gehalt an verfügbaren Glucan in der Bierhefesuspension BS.

Die Erfindung ist jedoch nicht auf das gezeigte Ausführungsbeispiel beschränkt. Dieses kann somit im Rahmen der Erfindung beliebig abgewandelt werden.

So können anstelle einer Bierhefesuspension beispielsweise auch andere Hefesuspensionen eingesetzt werden. Ebenso können nicht zwingende Verfahrensschritte, wie z.B. der Waschprozess 102, weggelassen werden. Des Weiteren kann der Zellaufschluss durch gepulste elektrische Felder statt mit einer Kugelmühle 5 erfolgen. Auch ist es unter anderem möglich, das Mannan und/oder das Glucan anstatt mit den beschriebenen Filterstufen (Mikrofilter 16 und Ultrafilter 18) mit einem Trommelfilter oder einer Zentrifuge abzutrennen.

Weiter kann vor dem Verdampfer 12 und/oder vor dem Verdampfer 20 optional ein Membrankontaktor MK bzw. MK' vorgesehen werden. Damit können die entsprechenden Phasen vor dem Sprühtrocknen mit einem Oxidationsmittel, beispielsweise Ozon und/oder Wasserstoffperoxid, behandelt werden.

Zusammenfassend ist festzustellen, dass ein neuartiges und besonders vorteilhaftes Verfahren zum Aufschliessen von Hefe, insbesondere Bierhefe, zur Isolierung von Proteinen und Sacchariden bereitgestellt wurde, welches sich insbesondere für grosstechnische Ansätze eignet.

## Patentansprüche

1. Verfahren zum Aufschliessen von Hefe, insbesondere Bierhefe, zur Isolierung von Proteinen und Sacchariden umfassend die Schritte:
a) Bereitstellen einer Hefesuspension, insbesondere einer Bierhefesuspension;
b) physikalisches Aufschliessen der Hefe in der Hefesuspension;
c) Mikrofiltration der aufgeschlossenen Hefesuspension aus Schritt b);
d) Ultrafiltration des Permeats der Mikrofiltration aus Schritt c) wobei als Retentat der Ultrafiltration eine Phase mit Proteinen als Hauptfeststoffbestandteil abgetrennt wird;
e) Behandeln des Retentats der Mikrofiltration aus Schritt c) mit einer Protease unter basischen Bedingungen und anschliessende Abtrennung einer Phase mit Mannan als Hauptfeststoffbestandteil, insbesondere durch Filtration und/oder Zentrifugieren;
f) Behandeln der in Schritt e) zurückgehaltenen Phase aufeinanderfolgend zuerst bei basischen und danach bei sauren Bedingungen und anschliessende Abtrennung einer Phase mit Glucan als Hauptfeststoffbestandteil, insbesondere durch Filtration und/oder Zentrifugieren.

2. Verfahren nach Anspruch 1, wobei die in Schritt a) bereitgestellte Hefesuspension bezogen auf das Gesamtgewicht der Hefesuspension einen Feststoffanteil von 5 - 30 Gew.-%, insbesondere 7 - 20 Gew.-%, aufweist.

3. Verfahren nach einem der vorangehenden Ansprüche, wobei die in Schritt a) bereitgestellte Hefesuspension eine Temperatur von 0 - 15°C, bevorzugt 2 - 12°C, im Besonderen 3 - 7°C oder 5°C, aufweist.

4. Verfahren nach einem der vorangehenden Ansprüche, wobei die Hefesuspension vor Schritt b) einem Waschprozess unterzogen wird bei welchem wenigstens ein Teil der flüssigen Phase der Hefesuspension, im Besonderen wenigstens 50 Gew.-%, im Speziellen wenigstens 60 Gew.-%, der flüssigen Phase der Hefesuspension, durch eine weitere Flüssigkeit, insbesondere Wasser, ausgetauscht wird.

5. Verfahren nach einem der vorangehenden Ansprüche, wobei das physikalische Aufschliessen in Schritt b) in einem Homogenisator oder einer Mahlkörpermühle, insbesondere eine Kugelmühle, erfolgt.

6. Verfahren nach einem der vorangehenden Ansprüche, wobei das physikalische Aufschliessen in Schritt b) durch gepulste elektrische Felder erfolgt.

7. Verfahren nach Anspruch 6, wobei die elektrische Feldstärke 1 - 30 kV/cm, insbesondere 5 - 24 kV/cm, beträgt und/oder der spezifische Energieeintrag 1 - 180 kJ/L, insbesondere 3 - 120 kJ/L, beträgt.

8. Verfahren nach einem der vorangehenden Ansprüche, wobei die Mikrofiltration in Schritt c) mit einem Filtermedium mit einer Porengrösse von 0.1 - 0.5 µm, bevorzugt 0.1 - 0.2 µm, insbesondere 0.1 µm, erfolgt und wobei die Ultrafiltration in Schritt d) mit einem Filtermedium mit einer Porengrösse von kleiner als 90 nm und/oder einer Ausschlussgrenze von 1 - 100 kDa, im Speziellen 5 - 100 kDa, bevorzugt 10 - 50 kDa, insbesondere 15 - 25 kDa, im Speziellen 20 kDa erfolgt.

9. Verfahren nach einem der vorangehenden Ansprüche, wobei die Mikrofiltration in Schritt c) und/oder die Ultrafiltration in Schritt d) als Diafiltration durchgeführt wird, wobei vorlagenseitig bevorzugt fortlaufend Wasser zugeführt wird.

10. Verfahren nach einem der vorangehenden Ansprüche, wobei die in Schritt d) abgetrennte Phase mit Proteinen als Hauptfeststoffbestandteil nach Schritt d) getrocknet, insbesondere sprühgetrocknet, wird.

11. Verfahren nach einem der vorangehenden Ansprüche, wobei in Schritt e) Protease mit einem Anteil von 0.0001-10 Gew.-%, insbesondere 0.001-5 Gew.-%, im Speziellen 0.01-1 Gew.-% oder 0.05-0.5 Gew.-%, bezogen auf den Feststoffgehalt des Retentats der Mikrofiltration aus Schritt c), verwendet wird, wobei es sich bei der Protease bevorzugt um ein Subtilisin handelt und wobei die Behandlung in Schritt e) bei einem pH-Wert von 7.5 - 13, bevorzugt 8 - 11, im Besonderen 8.5 - 10.5, und bei einer Temperatur von 40 - 80°C, bevorzugt 50 - 70°C, insbesondere 55 - 65°C, erfolgt.

12. Verfahren nach einem der vorangehenden Ansprüche, wobei die Behandlung mit Protease in Schritt e) 10 min bis 18 Stunden, insbesondere 3 - 12 Stunden, dauert.

13. Verfahren nach einem der vorangehenden Ansprüche, wobei in Schritt f) die zurückgehaltene Phase in einem ersten Zeitintervall, bevorzugt für 1 - 5 Stunden, bei einem pH-Wert von 7.5 - 13, bevorzugt 8 - 11, im Besonderen 8.5 - 10.5, und einer Temperatur von 40 - 95°C, bevorzugt 65 - 85°C, insbesondere 75 - 85°C, behandelt wird und anschliessend in einem zweiten Zeitintervall, bevorzugt für 0.5 - 2 Stunden, bei einem pH-Wert von 2 - 6.5, bevorzugt 3 - 5, im Besonderen 3.5 - 4.5, und einer Temperatur von 50 - 95°C, bevorzugt 80 - 95°C, insbesondere 80 - 90°C, behandelt wird.

14. Verfahren nach Anspruch 13, wobei in Schritt f) der pH-Wert im ersten Zeitintervall dem bei der Behandlung in Schritt e) vorliegenden pH-Wert entspricht und wobei in Schritt f) die Temperatur im ersten Zeitinterwall geringer ist als im zweiten Zeitintervall und/oder das erste Zeitintervall länger ist als das zweite Zeitintervall.

15. Verfahren nach einem der vorangehenden Ansprüche, wobei die Abtrennung der Phase mit Mannan als Hauptfeststoffbestandteil in Schritt e) und/oder die Abtrennung einer Phase mit Glucan als Hauptfeststoffbestandteil in Schritt f) durch Filtration, insbesondere eine kombinierte Mikrofiltration und Ultrafiltration erfolgt.

16. Verfahren nach Anspruch 15, wobei die Mikrofiltration mit einem Filtermedium mit einer Porengrösse von 0.1 - 0.5 µm, insbesondere 0.1 - 0.2 µm, bevorzugt 0.1 µm, erfolgt und wobei die Ultrafiltration mit einem Filtermedium mit einer Porengrösse von kleiner als 90 nm und/oder einer Ausschlussgrenze von 1 - 100 kDa, im Speziellen 5 - 100 kDa, bevorzugt 10 - 50 kDa, insbesondere 15 - 25 kDa, im Speziellen 20 kDa, erfolgt.

17. Verfahren nach einem der Ansprüche 15 - 16, wobei die Mikrofiltration in Schritt e) und/oder die Ultrafiltration in Schritt f) als Diafiltration durchgeführt wird.

18. Verfahren nach einem der vorangehenden Ansprüche, wobei die in Schritt e) abgetrennte Phase mit Mannan als Hauptfeststoffbestandteil nach Schritt e) getrocknet, insbesondere sprühgetrocknet, wird und/oder wobei die nach Schritt f) abgetrennte Phase mit Glucan als Hauptfeststoffbestandteil nach Schritt f) getrocknet, insbesondere sprühgetrocknet, wird.

## Claims

1. Method for digesting yeast, in particular brewer's yeast, for the isolation of proteins and saccharides comprising the steps:
a) Providing a yeast suspension, in particular a brewer's yeast suspension;
b) Physical digestion of the yeast in the yeast suspension;
c) Microfiltration of the digested yeast suspension from step b);
d) Ultrafiltration of the permeate of the microfiltration from step c) wherein a phase with proteins as the main solid component is separated as the retentate of the ultrafiltration;
e) Treating the retentate of the microfiltration from step c) with a protease under basic conditions and subsequently separating a phase with mannan as the main solid component, in particular by filtration and/or centrifugation;
f) treating the phase retained in step e) successively first at basic and then at acidic conditions and subsequently separating a phase with glucan as the main solid component, in particular by filtration and/or centrifugation.

2. Method according to claim 1, wherein the yeast suspension provided in step a) has a solids content of 5-30 % by weight, in particular 7-20 % by weight, based on the total weight of the yeast suspension.

3. Method according to any one of the preceding claims, wherein the yeast suspension provided in step a) has a temperature of 0 - 15°C, preferably 2 - 12°C, more particularly 3 - 7°C or 5°C.

4. Method according to one of the preceding claims, wherein the yeast suspension is subjected to a washing process before step b), in which at least part of the liquid phase of the yeast suspension, in particular at least 50% by weight, in particular at least 60% by weight, of the liquid phase of the yeast suspension, is replaced by another liquid, in particular water.

5. Method according to any one of the preceding claims, wherein the physical digestion in step b) is carried out in a homogenizer or a grinding media mill, in particular a ball mill.

6. Method according to any one of the preceding claims, wherein the physical digestion in step b) is performed by pulsed electric fields.

7. Method according to claim 6, wherein the electric field strength is 1 - 30 kV/cm, in particular 5 - 24 kV/cm, and/or the specific energy input is 1 - 180 kJ/L, in particular 3 - 120 kJ/L.

8. Method according to one of the preceding claims, wherein the microfiltration in step c) is carried out with a filter medium having a pore size of 0.1 - 0.5 µm, preferably 0.1 - 0.2 µm, in particular 0.1 µm, and wherein the ultrafiltration in step d) is carried out with a filter medium having a pore size of less than 90 nm and/or an exclusion limit of 1 - 100 kDa, preferably 10 - 50 kDa, in particular 15 - 25 kDa, in particular 20 kDa.

9. Method according to one of the preceding claims, wherein the microfiltration in step c) and/or the ultrafiltration in step d) is carried out as diafiltration, wherein water is preferably continuously supplied on the feed side.

10. Method according to any one of the preceding claims, wherein the phase separated in step d) with proteins as the main solid component is dried, in particular spray-dried, after step d).

11. Method according to one of the preceding claims, wherein in step e) protease is used in a proportion of 0.0001-10 % by weight, in particular 0.001-5 % by weight, more particularly 0.01-1 % by weight or 0.05-0.5 % by weight, based on the solids content of the retentate of the microfiltration from step c), wherein the protease is preferably a subtilisin and wherein the treatment in step e) is carried out at a pH of 7.5 - 13, preferably 8 - 11, in particular 8.5 - 10.5, and at a temperature of 40 - 80°C, preferably 50 - 70°C, in particular 55 - 65°C.

12. Method according to any one of the preceding claims, wherein the treatment with protease in step e) lasts 10 min to 18 hours, in particular 3 - 12 hours.

13. Method according to any one of the preceding claims, wherein in step f) the retained phase is treated in a first time interval, preferably for 1 - 5 hours, at a pH of 7.5 - 13, preferably 8 - 11, in particular 8.5 - 10. 5, and a temperature of 40 - 95°C, preferably 65 - 85°C, in particular 75 - 85°C, and subsequently treated in a second time interval, preferably for 0.5 - 2 hours, at a pH of 2 - 6.5, preferably 3 - 5, in particular 3.5 - 4.5, and a temperature of 50 - 95°C, preferably 80 - 95°C, in particular 80 - 90°C.

14. Method according to claim 13, wherein in step f) the pH in the first time interval is equal to the pH present during the treatment in step e), and wherein in step f) the temperature in the first time interval is lower than in the second time interval and/or the first time interval is longer than the second time interval.

15. Method according to one of the preceding claims, wherein the separation of the phase with mannan as the main solid component in step e) and/or the separation of a phase with glucan as the main solid component in step f) is carried out by filtration, in particular a combined microfiltration and ultrafiltration.

16. Method according to claim 15, wherein the microfiltration is carried out with a filter medium having a pore size of 0.1 - 0.5 µm, in particular 0.1 - 0.2 µm, preferably 0.1 µm, and wherein the ultrafiltration is carried out with a filter medium having a pore size of less than 90 nm and/or an exclusion limit of 1 - 100 kDa, preferably 10 - 50 kDa, in particular 15 - 25 kDa, in particular 20 kDa.

17. Method according to any one of claims 15 - 16, wherein the microfiltration in step e) and/or the ultrafiltration in step f) is carried out as diafiltration.

18. Method according to any one of the preceding claims, wherein the phase separated in step e) with mannan as the main solids component after step e) is dried, in particular spray-dried, and/or wherein the phase separated after step f) with glucan as the main solids component after step f) is dried, in particular spray-dried.

## Revendications

1. Procédé de digestion de levure, en particulier de levure de bière, pour l'isolement de protéines et de saccharides, comprenant les étapes suivantes :
a) préparation d'une suspension de levure, en particulier d'une suspension de levure de bière ;
b) digestion physique de la levure dans la suspension de levure ;
c) microfiltration de la suspension de levure digérée au cours de l'étape b) ;
d) ultrafiltration du perméat de la microfiltration de l'étape c), dans laquelle une phase contenant des protéines comme composant solide principal est séparée en tant que rétentat de l'ultrafiltration ;
e) traitement du rétentat de la microfiltration de l'étape c) avec une protéase dans des conditions basiques, puis séparation consécutive d'une phase contenant du mannane comme composant solide principal, en particulier par filtration et/ou centrifugation ;
f) traitement de la phase retenue au cours de l'étape e) d'abord dans des conditions basiques et ensuite dans des conditions acides, successivement, puis séparation consécutive d'une phase contenant du glucane comme composant solide principal, en particulier par filtration et/ou centrifugation.

2. Procédé selon la revendication 1, dans lequel la suspension de levure préparée au cours de l'étape a) présente une teneur en solides de 5 à 30 % en poids, en particulier de 7 à 20 % en poids, par rapport au poids total de la suspension de levure.

3. Procédé selon l'une des revendications précédentes, dans lequel la suspension de levure préparée au cours de l'étape a) présente une température de 0 à 15 °C, de préférence de 2 à 12 °C, en particulier de 3 à 7 °C ou de 5 °C.

4. Procédé selon l'une des revendications précédentes, dans lequel la suspension de levure est soumise, avant l'étape b), à un processus de lavage dans lequel au moins une partie de la phase liquide de la suspension de levure, en particulier au moins 50 % en poids, en particulier au moins 60 % en poids, de la phase liquide de la suspension de levure, est remplacée par un autre liquide, en particulier de l'eau.

5. Procédé selon l'une des revendications précédentes, dans lequel la digestion physique au cours de l'étape b) a lieu dans un homogénéisateur ou un moulin à corps de broyage, en particulier un broyeur à billes.

6. Procédé selon l'une des revendications précédentes, dans lequel la digestion physique au cours de l'étape b) est réalisée par le biais de champs électriques pulsés.

7. Procédé selon la revendication 6, dans lequel l'intensité de champ électrique est de 1 à 30 kV/cm, en particulier de 5 à 24 kV/cm, et/ou l'apport d'énergie spécifique est de 1 à 180 kJ/L, en particulier de 3 à 120 kJ/L.

8. Procédé selon l'une des revendications précédentes, dans lequel la microfiltration au cours de l'étape c) a lieu avec un milieu filtrant ayant une taille de pore de 0,1 à 0,5 µm, de préférence de 0,1 à 0,2 µm, en particulier de 0,1 µm, et dans lequel l'ultrafiltration au cours de l'étape d) a lieu avec un milieu filtrant ayant une taille de pore inférieure à 90 nm et/ou une limite d'exclusion de 1 à 100 kDa, en particulier de 5 à 100 kDa, de préférence de 10 à 50 kDa, en particulier de 15 à 25 kDa, en particulier de 20 kDa.

9. Procédé selon l'une des revendications précédentes, dans lequel la microfiltration au cours de l'étape c) et/ou l'ultrafiltration au cours de l'étape d) sont réalisées sous la forme d'une diafiltration, dans laquelle de l'eau est introduite de préférence en continu côté alimentation.

10. Procédé selon l'une des revendications précédentes, dans lequel la phase séparée au cours de l'étape d) avec des protéines comme composant solide principal est séchée après l'étape d), en particulier séchée par pulvérisation.

11. Procédé selon l'une des revendications précédentes, dans lequel la protéase est utilisée au cours de l'étape e) en une proportion de 0,0001 à 10 % en poids, en particulier de 0,001 à 5 % en poids, en particulier de 0,01 à 1 % en poids ou de 0,05 à 0,5 % en poids, par rapport à la teneur en solides du rétentat de la microfiltration de l'étape c), dans lequel la protéase est de préférence une subtilisine et dans lequel le traitement au cours de l'étape e) a lieu à un pH de 7,5 à 13, de préférence de 8 à 11, en particulier de 8,5 à 10,5, et à une température de 40 à 80 °C, de préférence de 50 à 70 °C, en particulier de 55 à 65 °C.

12. Procédé selon l'une des revendications précédentes, dans lequel le traitement avec la protéase au cours de l'étape e) dure de 10 min à 18 heures, en particulier de 3 à 12 heures.

13. Procédé selon l'une des revendications précédentes, dans lequel la phase retenue au cours de l'étape f) est traitée au cours d'un premier intervalle de temps, de préférence pendant 1 à 5 heures, à un pH de 7,5 à 13, de préférence de 8 à 11, en particulier de 8,5 à 10,5, et à une température de 40 à 95 °C, de préférence de 65 à 85 °C, en particulier de 75 à 85 °C, et est ensuite traitée au cours d'un deuxième intervalle de temps, de préférence pendant 0,5 à 2 heures, à un pH de 2 à 6,5, de préférence de 3 à 5, en particulier de 3,5 à 4,5, et à une température de 50 à 95 °C, de préférence de 80 à 95 °C, en particulier de 80 à 90 °C.

14. Procédé selon la revendication 13, dans lequel, au cours de l'étape f), le pH durant le premier intervalle de temps correspond au pH présent lors du traitement de l'étape e) et dans lequel, au cours de l'étape f), la température durant le premier intervalle de temps est inférieure à celle durant le deuxième intervalle de temps et/ou le premier intervalle de temps est plus long que le deuxième intervalle de temps.

15. Procédé selon l'une des revendications précédentes, dans lequel la séparation de la phase contenant du mannane comme composant solide principal au cours de l'étape e) et/ou la séparation d'une phase contenant du glucane comme composant solide principal au cours de l'étape f) a lieu par filtration, en particulier une microfiltration et une ultrafiltration combinées.

16. Procédé selon la revendication 15, dans lequel la microfiltration a lieu avec un milieu filtrant ayant une taille de pore de 0,1 à 0,5 µm, en particulier de 0,1 à 0,2 µm, de préférence de 0,1 µm, et dans lequel l'ultrafiltration a lieu avec un milieu filtrant ayant une taille de pore inférieure à 90 nm et/ou une limite d'exclusion de 1 à 100 kDa, en particulier de 5 à 100 kDa, de préférence de 10 à 50 kDa, en particulier de 15 à 25 kDa, en particulier de 20 kDa.

17. Procédé selon l'une des revendications 15 à 16, dans lequel la microfiltration au cours de l'étape e) et/ou l'ultrafiltration au cours de l'étape f) sont réalisées sous la forme d'une diafiltration.

18. Procédé selon l'une des revendications précédentes, dans lequel la phase séparée au cours de l'étape e) avec du mannane comme composant solide principal selon est séchée après l'étape e), en particulier séchée par pulvérisation, et/ou dans lequel la phase séparée au cours de l'étape f) avec du glucane comme composant solide principal est séchée après l'étape f), en particulier séchée par pulvérisation.
